# EUROPEAN PATENT SPECIFICATION

(11) **EP 3 447 480 B1**
(45) Date of publication and mention of the grant of the patent: **09.03.2022**
(21) Application number: 18185677.4
(22) Date of filing: 26.07.2018
(51) Int. Cl.: G01N 25/72, G01N 33/44, B29C 70/30, B29C 70/54, G06T 7/00, G01N 21/84, G01N 21/88, G01N 33/00, G01J 5/00

(54) **IN-PROCESS MONITORING; AUTOMATED DECISION-MAKING; AND PROCESS CONTROL FOR COMPOSITE MANUFACTURING USING PART-REFERENCED PLY-BY-PLY INFRARED THERMOGRAPHY AND OTHER NON-CONTACT NON-DESTRUCTIVE INSPECTION**
PROZESSINTERNE ÜBERWACHUNG, AUTOMATISIERTE ENTSCHEIDUNGSFINDUNG UND PROZESSSTEUERUNG BEI DER HERSTELLUNG VON VERBUNDWERKSTOFFEN MITTELS TEILREFERENZIERTER SCHICHT-FÜR-SCHICHT-INFRAROT-THERMOGRAFIE UND ANDEREN BERÜHRUNGSLOSEN, ZERSTÖRUNGSFREIEN PRÜFUNGEN
SURVEILLANCE EN COURS, PRISE DE DÉCISION AUTOMATISÉE ET COMMANDE DE PROCESSUS POUR LA FABRICATION COMPOSITE À L'AIDE D'UNE THERMOGRAPHIE INFRAROUGE DE RÉFÉRENCE DE PIÈCES PLI PAR PLI ET AUTRE INSPECTION NON DESTRUCTIVE SANS CONTACT

(30) Priority: 24.08.2017 US 201715685684
(43) Date of publication of application: 27.02.2019
(73) Proprietor: The Boeing Company, Chicago, IL 60606-1596 (US)
(72) Inventor: THOMPSON, Jeffrey G., Chicago, IL 60606-1596 (US); GEORGESON, Gary E., Chicago, IL 60606-1596 (US); BINGHAM, Jill P., Chicago, IL 60606-1596 (US)
(74) Representative: Witte, Weller & Partner Patentanwälte mbB

(56) References cited:
- EP-A1- 3 179 241
- EP-A2- 3 007 022
- WO-A1-2006/093703

## Description

### BACKGROUND

Aspects described herein relate to flaw detection in composite laminates, and more specifically, to detecting flaws in green composite materials, which are composite materials that have not yet been cured.

Document EP 3 179 241 A1, according to its abstract, states a method and an apparatus for detecting defects in a laminate of uncured, compacted composite sheets. After a number of plies of composite sheets are arranged and compacted, a burst of heat energy is applied to a top surface of the laminate and a digital thermographic camera captures images of the top surface. A computer processor measures heat characteristics of the top surface to identify regions of the top surface with different heat characteristics. Such different areas are identified as regions that include a defect. The defect regions can be repaired by applying additional compaction and/or by removing at least a portion of some layers, removing any foreign object debris, replacing the layers, and compacting the replaced layers. After any defects are addressed, the laminate is cured.

Document WO 2006/093703 A1, according to its abstract, states a real-time thermal imaging apparatus and method includes multiple digital infrared cameras mounted to an automated composite material layup device to record digital infrared images of the composite material surface on a real-time basis during a composite material layup process. The digital infrared cameras are triggered periodically to produce digital images of the composite material. The digital image data is sent to an image analyzer which detects edges of and anomalies in the composite material and generates alarm and other process control signals. The image analyzer also aggregates the digital images from the multiple cameras and the digital images recorded over a sequence in time to produce a continuous virtual digital image of the composite material surface. The digital image data and associated analysis results are saved and may be displayed on a real-time basis or at a later time.

Document EP 3 007 022 A2, according to its abstract, states that a method, system and computer-readable storage medium are provided to facilitate inspection of a composite part during manufacture. In the context of a system, a system for inspecting a composite part during manufacture is provided that includes an inspection system configured to detect an in-process anomaly with respect to a ply of the composite part during placement of the ply. The system also includes a computing system configured to determine part location coordinates of the in-process anomaly detected by the inspection system with respect to the ply of the composite part. The computing system is also configured to map the in-process anomaly to a digital part model based upon the part location coordinates. The system additionally includes a display, responsive to the computing system, configured to present a representation of the digital part model including an indication of the in-process anomaly relative thereto.

### SUMMARY

There is provided a method for detecting a defect in a workpiece, wherein the workpiece comprises a plurality of plies; wherein, for each of the plurality of plies, the following steps are conducted: laying a ply of material; applying thermal energy to a surface of the ply of material; capturing at least one digital thermographic image of the surface of the ply of material after applying the thermal energy; using a computer processor, analyzing at least one heat characteristic of regions of the ply of material in the at least one digital thermographic image to identify defect regions in which the at least one heat characteristic is different than baseline data, wherein analyzing the at least one heat characteristic of regions of the ply of material in the at least one digital thermographic image comprises determining whether a rejectable flaw exists and determining whether a sub-rejectable flaw exists; wherein, when it is determined that a rejectable flaw exists, the following steps are performed: halting processing; outputting an alert; repairing the rejectable flaw; and resuming processing; wherein, when it is determined that a sub-rejectable flaw exists, the following steps are performed: recording one or more of the location, type, and extent of the sub-rejectable flaw; and resuming processing; wherein the method further comprises: determining whether a final ply of the workpiece has been laid; and laying a further ply of material on the ply of material, if the ply is not the final ply of the workpiece.

There is further provided a system for detecting a defect in a workpiece, wherein the workpiece comprises a plurality of plies, comprising: a work surface for arranging and compacting uncured composite layers; a heat source arranged relative to the surface and operable to provide thermal energy to a surface of a ply of material on the work surface; a thermographic digital camera arranged relative to the surface and operable to capture at least one digital thermographic image of the surface of the ply of material; a computer processor in communication with the thermographic digital camera and the heat source; and a computer memory containing a program that, when executed on the computer processor, performs, for each of the plurality of plies, an operation for processing data, comprising: applying thermal energy to a surface of the ply of material; capturing at least one digital thermographic image of the surface of the ply of material after applying the thermal energy; analyzing at least one heat characteristic of regions of the ply of material in the at least one digital thermographic image to identify defect regions in which the at least one heat characteristic is different than baseline data, wherein analyzing the at least one heat characteristic of regions of the ply of material in the at least one digital thermographic image comprises determining whether a rejectable flaw exists and determining whether a sub-rejectable flaw exists; wherein, when it is determined that a rejectable flaw exists, the following steps are performed: halting processing; outputting an alert; repairing the rejectable flaw; and resuming processing; wherein, when it is determined that a sub-rejectable flaw exists, the following steps are performed: recording one or more of the location, type, and extent of the sub-rejectable flaw; and resuming processing; wherein the program, when executed on the computer processor, performs an additional operation for processing data, comprising: determining whether a final ply of the workpiece has been laid; and laying a further ply of material on the ply of material after resuming processing, if the ply is not the final ply of the workpiece.

### BRIEF DESCRIPTION OF THE SEVERAL VIEWS OF THE DRAWINGS

Figure 1A is a perspective view of a layup table with four plies of composite sheets of a laminate laid out on a surface of the table, wherein three of the four plies are shown partially cut away to display the underlying plies;
Figure 1B is a cross-sectional side view of the four plies of the laminate of Figure 1A with a disbond between the second and third plies;
Figure 1C is a cross-sectional side view of the four plies of the laminate of Figure 1A with an inclusion between the second and third plies;
Figure 2 is a block diagram of a system for detecting defects in a laminate of uncured, compacted composite sheets, wherein the system is shown detecting defects in the laminate shown in Figure 1A as an exemplary scenario;
Figure 3 is a flow chart of a process for detecting a defect in a laminate of uncured, compacted composite sheets;
Figure 4 is a flow chart of a process for building a laminate of compacted composite sheets;
Figure 5A is a chart illustrating a temperature response over time for a defect-free laminate of uncured, compacted composite sheets and a laminate of uncured, compacted composite sheets having a conductive inclusion;
Figure 5B is a chart illustrating a temperature response over time for a defect-free laminate of uncured, compacted composite sheets and a laminate of uncured, compacted composite sheets having a disbond and/or an inclusion;
Figure 6A is a chart illustrating a first derivative of the temperature response over time for the defect-free laminate of uncured, compacted composite sheets and the laminate of uncured, compacted composite sheets having the conductive inclusion shown in Figure 5A;
Figure 6B is a chart illustrating a first derivative temperature response over time for the defect-free laminate of uncured, compacted composite sheets and the laminate of uncured, compacted composite sheets having the disbond and/or insulative inclusion shown in Figure 5B;
Figure 7A is a chart illustrating a second derivative of the temperature response over time for the defect-free laminate of uncured, compacted composite sheets and the laminate of uncured, compacted composite sheets having the conductive inclusion shown in Figure 5A;
Figure 7B is a chart illustrating a second derivative temperature response over time for the defect-free laminate of uncured, compacted composite sheets and the laminate of uncured, compacted composite sheets having the disbond and/or insulative inclusion shown in Figure 5B; and
Figure 8 is a flow chart of a process for detecting a defect, according to another aspect of the disclosure.

### DETAILED DESCRIPTION

Structures, such as wing spars and fuselage barrel sections of aircraft can be made with composite materials. The composite materials are typically formed as a laminate of several plies of composite sheets. After the plies of composite sheets are laid out, the resulting laminate is cured. After curing, the laminate is checked for defects, such as disbonds (i.e., regions in which two adjacent plies do not bond together resulting in a void) and inclusions (i.e., regions in which foreign object debris is between two adjacent plies). To correct such defects, the region with the defect is scarfed out to remove the defect and a patch of plies is installed in the scarfed out area. The patch is then cured and the rechecked for any additional defects. Such a repair procedure can be expensive and time consuming.

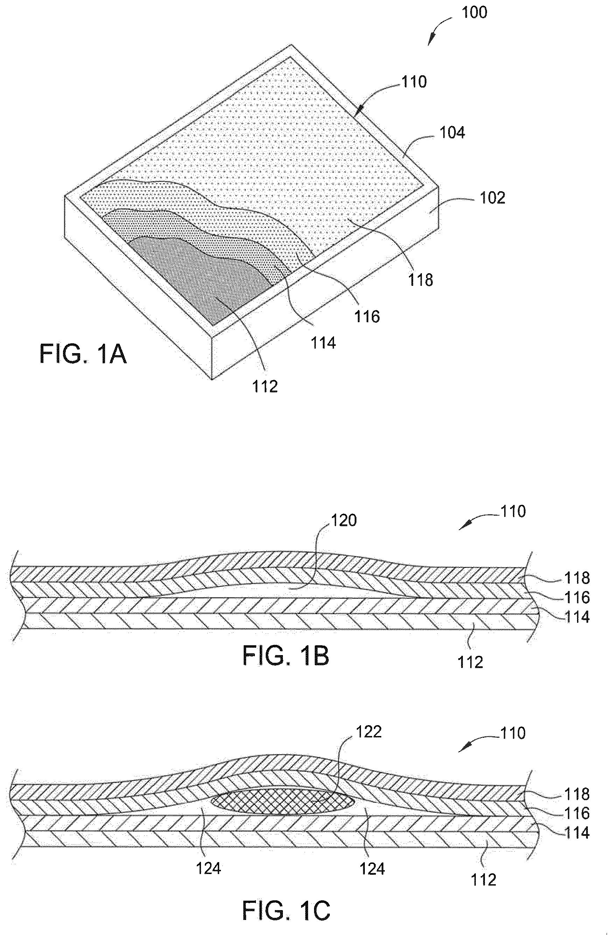

Figure 1A is a perspective view of a layup table 102 with a laminate 110 that includes four plies of composite sheets 112, 114, 116, and 118 arranged on a work surface 104 of the layup table 102. The top three plies of composite sheets 114, 116, and 118 are shown in partial cutaway to illustrate the underlying plies. The four plies are arranged in alternating directions. The first ply 112 includes fibers (e.g., carbon fibers) arranged in a first direction, the second ply 114 includes fibers arranged in a second direction that is orthogonal to the first direction, the third ply 116 includes fibers arranged in the first direction, and the fourth play includes fibers arranged in the second direction. In practice, a person or a machine would lay the first ply 112 on the work surface 104 of the layup table 102 with the fibers of the first ply 112 arranged in the first direction. The person or machine would then lay the second ply 114 on the first ply 112 with the fibers of the second ply 114 arranged in the second direction. The person or machine would then lay the third play 116 on the second ply 114 with the fibers of the third ply 116 arranged in the first direction. The person or machine would then lay the fourth ply 118 on the third ply 116 with the fibers of the fourth ply 118 arranged in the second direction. After the plies 112, 114, 116, and 118 have been laid on the work surface 104, the plies 112, 114, 116, and 118 can be compacted using rollers or the like. In various circumstances, additional plies could be laid on top of the illustrated four plies 112, 114, 116, and 118 and compacted. Also, in various circumstances, the number of plies that are laid down before a compaction step can be more or fewer than the illustrated four plies.

After compaction, it is possible for defects to exist in the uncured, compacted composite sheets. Figure 1B illustrates an example of a disbond 120. As shown in Figure 1B, the disbond 120 is formed where the second ply 114 and the third ply 116 were not compacted together. If a disbond 120 occurs, then the second ply 114 and the third ply 116 will not adhere to one another during a subsequent curing process. Figure 1C illustrates an example of an inclusion 122, in which a foreign object is arranged between plies. As shown in Figure 1C, the inclusion 122 is between the second ply 114 and the third ply 116. The inclusion could be a piece of backing paper for the second ply 114 or the third play 116 that was not removed, for example. The inclusion 122 can form disbonds 124 along edges of the inclusion 122 where the inclusion 122 prevents the adjacent plies (e.g., plies 114 and 116) from joining together. If the inclusion 122 is not removed, then the second ply 114 and the third ply 116 will not adhere to one another during the subsequent curing process. In various instances, the inclusion could be a conductive inclusion, which means that the inclusion transfers heat at a faster rate than the plies 112, 114, 116, and 118 of the laminate 110. For example, a conductive inclusion could be a metallic particle, such as a metal foil. In various other instances, the inclusion could be an insulative inclusion, which means that the inclusion transfers heat at a slower rate than the plies 112, 114, 116, and 118 of the laminate 110. For example, an insulative inclusion could be a piece of backing paper. In various instances, the inclusion could be a neutral inclusion, which means that the inclusion transfers heat at the same rate as the plies 112, 114, 116, and 118 of the laminate 110. For example, a neutral inclusion could be a piece of another ply.

Laminates, such as the laminate 110 shown in Figures 1A-1C, are currently inspected after the curing process using ultrasound. If a defect is found, the region of the cured laminate is scarfed out to remove the defect. Typically, the scarfed out region has a diameter of at least twenty times the size of the defect. Thus, a defect that is 0.1 centimeters across would be repaired with a scarfed out region having a diameter of two centimeters, for example. After the defect has been scarfed out of the cured laminate, a patch of uncured composite sheets is fit into the scarfed out region, compacted, and then cured in place. The repaired area is then inspected again to check for additional defects. Such a repair process is time consuming, especially if multiple defects are found in a laminate.

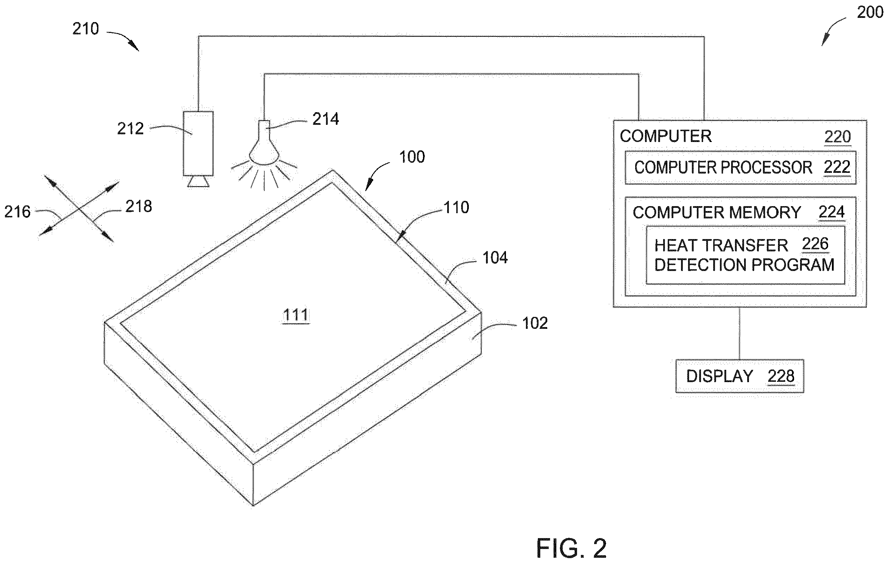

Figure 2 illustrates a system 200 for detecting defects in a laminate, such as disbonds and inclusions, before the laminate is cured. For purposes of illustration, the system 200 is described with reference to the laminate 110 discussed above with reference to Figures 1A-1C. However, the system 200 could be used on other laminates. If a defect is detected prior to the laminate 110 being cured, the defect can be repaired faster and cheaper. For example, a region that includes a detected disbond could be compacted again to remove the disbond. As another example, the plies could be separated in a region that includes a detected inclusion to remove the inclusion. The plies could then be put back in place and compacted again. Such repairs could be performed more quickly and less expensively than repairs after the laminate 110 is cured.

The system 200 includes a heat source 214, such as a heat lamp or a heat gun. The heat source 214 applies a burst of heat (i.e., a momentary heat load) to a top surface 111 of the laminate 110 to increase the temperature of the top surface 111 of the laminate 110 by a small amount. For the laminate 110 described in greater detail above, the heat source 214 would apply to the top-facing surface of the fourth ply 118. For example, the heat source 214 may apply heat to the top surface 111 of the laminate 110 to increase the temperature of the top surface 111 by a few degrees or less. In any case, the heat source 214 does not apply enough heat to cause the laminate 110 to begin to cure. Depending on the size of the laminate 110, the heat source 214 could be an array of heat sources arranged over the laminate 110 to provide uniform heat application to the top surface 111 of the laminate 110. As described in greater detail below, in various aspects, the heat source 214 could be movable in the directions of arrows 216 and 218 to apply heat to different locations along the top surface 111 of the laminate 110.

The system also includes a digital thermographic camera 212 that captures images of thermal properties (e.g., temperature) and changes to the thermal properties of the top surface 111 of the laminate 110 after the heat source 214 applies the momentary heat load. For example, the digital thermographic camera 212 could be an infrared camera. After heat is applied to the top surface 111 of the laminate 110 by the heat source 214, the heat travels from the top surface 111 through the laminate 110. The rate of heat transfer from the top surface 111 to internal layers of the laminate 110 is affected by defects, such as the disbonds and inclusions discussed above with reference to Figures 1B and 1C, which obstruct the flow of heat from the top surface 111 into the laminate 110. By capturing successive images of heat characteristics of the top surface 111 of the laminate 110 after the application of heat by the heat source 214, disbonds and inclusions (and other flaws) can be discovered. For example, the heat characteristics of the top surface 111 could be temperatures of the surface at different regions and/or different times, a first derivative of the temperature (i.e., rate of change of the temperature of the top surface 111) and/or a second derivative of the temperature (i.e., rate of change of the rate of change of the temperature of the top surface 111). In various aspects, the digital thermographic camera 212 could be movable in the directions of arrows 216 and 218. In certain aspects, the digital thermographic camera 212 and the heat source 214 move in the directions of arrows 216 and 218 together.

The heat source 214 and thermographic camera 212 are in communication with a computer 220. The computer 220 includes a computer processor 222 and computer memory 224. The digital thermographic camera 212 can transmit captured images of thermal properties of the top surface 111 of the laminate 110 to the computer 220 for storage on the computer memory 224. In operation, the computer processor 222 sends control signals to the heat source 214 to trigger the momentary heat output to the top surface 111 of the laminate 110. The computer processor 222 also sends control signals to the digital thermographic camera 212 to trigger the digital thermographic camera 212 to capture images of thermal properties of the top surface 111 laminate 110. The computer 220 can control movement of the heat source 214 and/or the digital thermographic camera 212 in the directions of arrows 216 and 218.

The computer memory 224 can also store a heat transfer detection program 226 that, when executed by the computer processor 222, triggers the digital thermographic camera 212 and the heat source 214 and processes the stored images of thermal properties of the top surface 111 of the laminate 110. The heat transfer detection program 226 can determine heat characteristics at the different locations of the top surface 111 of the laminate 110. The heat characteristics include at least one of temperatures at different locations of the top surface 111 of the laminate 110, a rate of change of temperatures (first derivative of temperature) at the different locations of the top surface 111 of the laminate 110, and a rate of change of the rate of change of temperatures (second derivative of temperature) at the different locations of the top surface 111 of laminate 110. The temperature, first derivative of temperature, and/or second derivative of temperature for the top surface 111 of the laminate 110 can be determined over a period of time to identify regions of the top surface 111 of the laminate 110 that have different heat transfer properties from the remainder of the laminate 110. As discussed, disbonds and/or inclusions (and other defects) in the laminate 110 can change the rate of heat transfer relative to regions of the laminate 110 that do not include such defects. For a typical laminate (e.g., laminate 110) that may have only a few defects, the majority of the top surface of the laminate will have the same or very similar heat characteristics. The regions of the laminate that include defects will have different heat characteristics. By identifying regions of the laminate 110 where the heat characteristics of the top surface 111 differ from other regions, the locations of defects can be identified.

The system 200 can include a display 228 in communication with the computer 220. In the event a defect is detected in the laminate 110 by the computer 220, the display 228 could output an alert or alarm. The display 228 and could provide an indication of a location of the laminate 110 that includes the defect. For example, the display 228 could be a computer display screen that flashes to indicate a detected defect and that provides coordinates and/or a graphical depiction of a location of the defect on the laminate 110.

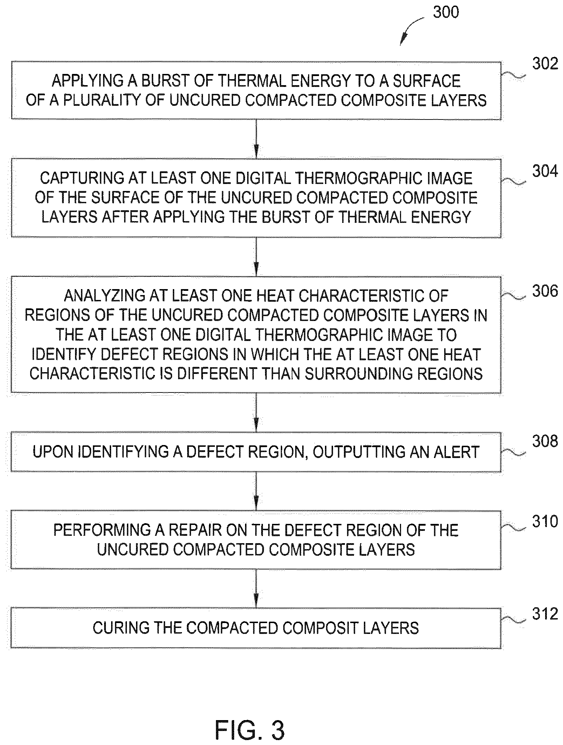

Figure 3 is a flowchart for a process 300 for detecting and correcting defects in a laminate (e.g., the laminate 110) prior to curing the laminate. In block 302, a burst of thermal energy is applied to a surface of a plurality of uncured compacted composite layers (e.g., the composite sheets 112, 114, 116, and 118 shown in Figures 1A-1C). As discussed above, the burst of thermal energy can be applied by the heat source 214 of the system 200, shown in Figure 2. In block 304, at least one digital thermographic image of the surface of the uncured compacted composite layers is captured. As discussed above, the least one digital thermographic image captured by the digital thermographic camera 212 of the system 200, shown in Figure 2. In block 306, the at least one digital thermographic image can be analyzed to identify at least one heat characteristic of regions of the uncured compacted composite layers to identify defect regions in which the at least one heat characteristic is different than surrounding regions. As discussed above, the heat transfer detection program 226 executing on the computer processor 222 of the system could analyze the at least one digital thermographic image stored in computer memory 224 to identify at least one heat characteristic, which could be a temperature over time, a first derivative of temperature over time, and/or a second derivative of temperature over time of the top surface 111 of the laminate 110. In block 308, upon identifying a defect region, and alarm or alert is output. As discussed above, the display 228 of the system 200 could display such an alarm or alert. In block 310, a repair is performed on the defect region of the uncured compacted composite layers. For example, if the defect in the defect region is known to be or believed to be a disbond, then additional compaction steps could be performed to eliminate the disbond. As another example, if the defect in the defect region is known to be or believed to be an inclusion, then layers of the laminate 110 can be at least partially removed to reveal the inclusion, the inclusion could be removed, and the layers could be replaced. Thereafter, an additional compaction procedure could be performed to re-compact the layers that had been removed. In block 312, after detected defects have been repaired, the laminate 110 can be cured.

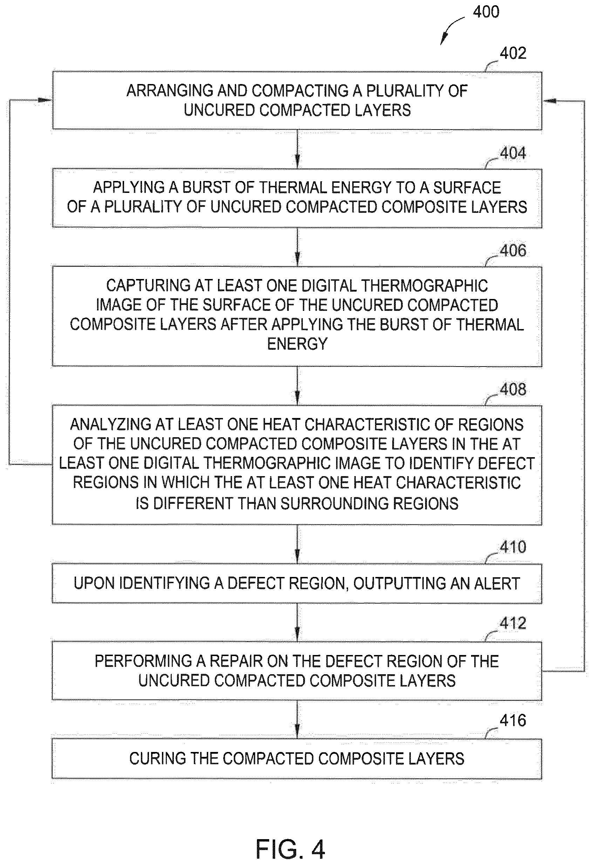

Figure 4 is a flowchart for a process 404 assembling a laminate that includes a plurality of layers. In block 402, a plurality of uncured composite layers are arranged and compacted. In block 404, a burst of thermal energy is applied to a surface of a plurality of uncured compacted composite layers (e.g., the composite sheets 112, 114, 116, and 118 shown in Figures 1A-1C). As discussed above, the burst of thermal energy can be applied by the heat source 214, shown in Figure 2. In block 406, at least one digital thermographic image of the surface of the uncured compacted composite layers is captured. As discussed above, the least one digital thermographic image captured by the digital thermographic camera 212, shown in Figure 2. In block 408, the at least one digital thermographic image can be analyzed to identify at least one heat characteristic of regions of the uncured compacted composite layers to identify defect regions in which the at least one heat characteristic is different than surrounding regions. As discussed above, the at least one heat characteristic could be a temperature over time, a first derivative of temperature over time, and/or a second derivative of temperature over time of the top surface 111 of the laminate 110. In the event no defects are found, then the process 400 could return to block 402, if necessary, to arrange and compact additional uncured composite layers on the laminate. Blocks 402, 404, 406, and 408 could be repeated until the laminate includes a suitable number of uncured composite layers. The number of uncured composite layers added with each iteration of blocks 402, 404, 406, and 408 of the process 400 can vary depending on various circumstances. In some circumstances, as few as one or two layers may be added with each iteration through blocks 402, 404, 406, and 408. In other circumstances, as many as four, five, or more uncured composite layers could be added with each iteration through blocks 402, 404, 406, and 408. As discussed in greater detail below, certain heat sources and digital thermographic cameras may have sufficient sensitivity to detect defects that are three, four, or more layers deep in a laminate of uncured, compacted composite layers whereas other heat sources and digital thermographic cameras may be limited to detecting defects that are one or two layers deep. Thus, the number of layers added with each iteration through blocks 402, 404, 406, and 408 could be dependent on the type of heat source and/or digital thermographic camera used in blocks 404 and 406.

In at least one aspect, blocks 402, 404, 406, and 408 could be performed in a continuous manner. For example, in at least one aspect, the laminate could be applied around a cylindrical mandrel by wrapping a continuous composite sheet around the mandrel. As the continuous composite sheet is laid on the mandrel (and on top of portions of the continuous sheet that have already been laid on the mandrel), the composite sheet is compacted (e.g., by a roller). The heat source and digital thermographic camera could follow behind the compacting apparatus to analyze the compacted uncured laminate for defects.

In block 410, upon identifying a defect region, and alarm or alert is output. In block 412, a repair is performed on the defect region of the uncured compacted composite layers. For example, if the defect in the defect region is known to be or is most likely a disbond, then additional compaction steps could be performed to eliminate the disbond. As another example, if the defect in the defect region is known to be or is most likely an inclusion, then layers of the laminate 110 can be removed to reveal the inclusion, the inclusion could be removed, and the layers could be replaced. Thereafter, additional compaction procedure could be performed to re-compact the layers that had been removed. After the defects have been removed, the process 400 can optionally return to block 402 to arrange and compact additional uncured composite layers.

In block 416, after all of the uncured composite layers have been arranged in compacted and any detected defects have been repaired, the laminate 110 can be cured.

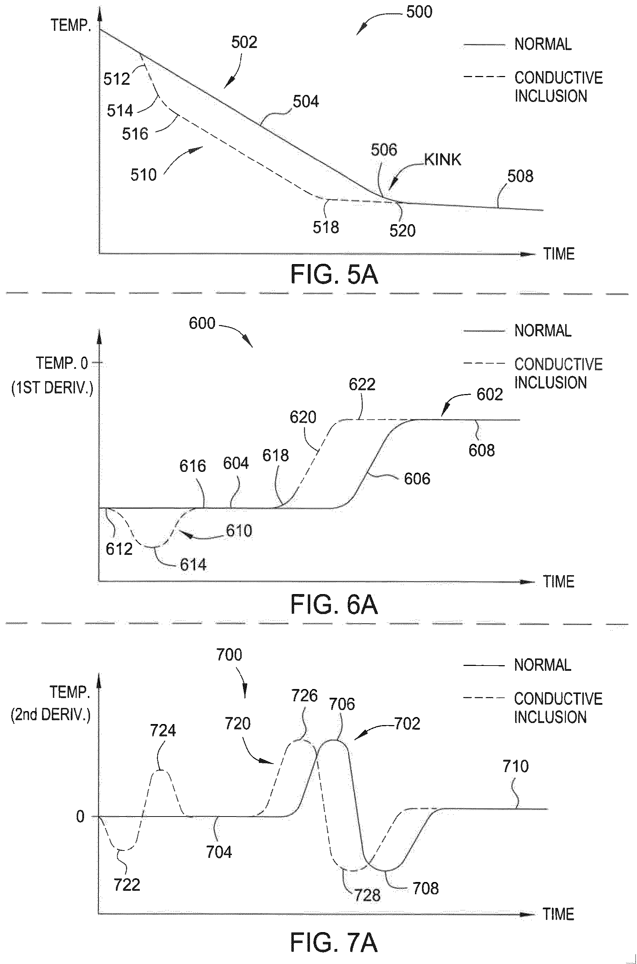

Figures 5A-5B illustrate temperature profiles that may be detected by a digital thermographic camera, such as the digital thermographic camera 212 shown in Figure 2. Figure 5A illustrates the differences in temperature of the top surface 111 of the laminate 110 over time for a defect-free region of the laminate and a region that has a conductive inclusion. As explained above, a conductive inclusion is an inclusion (e.g., debris) that conducts heat more readily than the laminate layers. For example, a piece of metal caught between layers of the laminate could be a conductive inclusion. After a heat source, such as the heat source 214 shown in Figure 2, outputs a burst of energy directed toward the top surface 111 of the laminate 110, the temperature of the top surface 111 of the laminate 110 rises. The chart 500 in Figure 5A includes a solid line 502 indicating the normal (i.e., defect free) region of the laminate and a broken line 510 indicating the region that has a conductive inclusion. The left-hand ends of the solid line 502 and the broken line 510 indicate the temperature of the top surface 111 of the laminate 110 immediately after the burst of energy from the heat source 214 is applied to the top surface 111. Energy absorbed by the top surface 111 has caused the temperature of the top surface 111 to increase. After the initial increase in temperature of the top surface 111, the temperature of the top surface 111 begins to decrease in a linear manner as the heat energy applied to the top surface 111 travels deeper into the laminate via conduction (as indicated by first portion 504 of the solid line 502). The initial rate of heat transfer away from the top surface 111 via conduction is substantially the same for both the normal region and the region that includes the inclusion, as indicated by the initial overlap of the solid line 502 and the broken line 510.

Referring to the normal region, indicated by the solid line 502, as the heat energy reaches a bottom side of the laminate 110, the heat energy can travel no further through the laminate via conductance. Thereafter, the heat transfer away from the top surface 111 slows, as indicated by the kink 506 and the reduced-slope second portion 508 of the solid line 502..

Referring to the conductive inclusion region, indicated by the broken line 510, before the heat energy reaches the bottom side of the laminate 110, the heat energy encounters the conductive inclusion (e.g., the inclusion 122 shown in Figure 1C could be a conductive inclusion). The exemplary conductive inclusion provides additional mass that can act as a heat sink for the laminate such that heat from the top surface 111 can be transferred to the inclusion via conductance. Furthermore, because the exemplary conductive inclusion is more conductive than the surrounding layers of laminate, the top surface 111 of the laminate 110 over the conductive inclusion cools faster than surrounding regions of the top surface 111. This faster cooling of the top surface 111 is indicated by a first kink 512 of the broken line 510 as the broken line 510 deviates from the solid line 502. As the conductive inclusion warms, the heat transfer from the top surface 111 slows, and the broken line 510 has a second kink 514. After the second kink 514, the rate of temperature change of the top surface 111 over the conductive inclusion is substantially equal to the rate of temperature change of the top surface 111 over normal regions of the laminate 110, as indicated by portion 504 of the solid line 502, as indicated by the portion 516 of the broken line 510. Because the conductive inclusion transfers heat away from the top surface 111 faster, the heat reaches the back surface of the laminate 110 at the conductive inclusion faster than at surrounding normal regions. Thereafter, the heat transfer away from the top surface 111 over the conductive inclusion slows, as indicated by the kink 518 in the broken line, which eventually joins the second portion of the solid line 502 (at the point indicated by reference numeral 520).

For a laminate that is only three, four, or five layers thick, the kinks 506 and 518 in the temperature profiles for a normal region and a region that includes a conductive inclusion may be observable in a reasonable period of time. For example, the kinks 506 and 518 may occur within three to ten seconds after heat is applied to the top surface 111 of the laminate 110. However, in instances in which the laminate is thicker (e.g., including tens of layers, such as fifty layers, one hundred layers, or one hundred and fifty layers), the kinks 506 and 518 may not be observable in a suitable period of time for such an inspection. In such instances, the kink 512 and/or the kink 514 in the temperature profile of the top surface 111 over the conductive inclusion should be perceivable. The kink 506 in the temperature profile of the top surface 111 over normal regions does not need to be observed. For example, assuming that most of the laminate 110 is free from defects, the temperature profile over time would be substantially homogenous. However, if a particular region includes a conductive inclusion, that region would have a temperature profile that decreases at a faster rate (at the kink 512) than the defect free regions. Thus, observing a temperature profile decreasing at a faster rate than surrounding regions could indicate the presence of a conductive inclusion in the laminate 110.

Figure 6A illustrates a chart 600 showing a calculated first derivative of the temperature (i.e., a rate of change of temperature) of the top surface 111 of laminate 110 for the defect free (i.e., normal) region, indicated by the solid line 602, and of the region that has the conductive inclusion, discussed above with reference to Figure 5A, indicated by the broken line 610. As shown in Figure 6A, the first derivative of temperature for the normal region is a first rate of change, indicated by portion 604 of the solid line 602, which corresponds with the first portion 504 of the solid line 502 in Figure 5A. Since the temperature of the top surface 111 is decreasing, the values of the first derivative are negative. Stated differently, a zero value on the temperature axis of the chart 600 is above the illustrated solid line 602 and the broken line 610. When the temperature profile of the top surface 111 over the normal region reaches the kink 506 in Figure 5A, the first derivative of temperature for the normal region increases (toward zero), as indicated by the portion 606 of the solid line 602. Thereafter, the first derivative of temperature for the normal region settles at a second rate of heat transfer, as indicated by the portion 608 of the solid line 602. The first derivative of temperature for the region that includes the conductive inclusion diverges from the first derivative of temperature for the normal region twice. First, where the temperature profile of the top surface 111 for the conductive inclusion decreases faster than surround normal regions at the kinks 512 and 514 (in Figure 5A), the first derivative of temperature decreases (away from zero) from the first rate of change (indicated by the portion 604 of the solid line 602) at the point indicated by reference numeral 612, reaches a peak at the point indicated by reference numeral 614, and then increases (toward zero) at the point indicated by reference numeral 616. When the temperature profile of the top surface 111 over the conductive inclusion reaches the kink 518 in Figure 5A, the first derivative of temperature increases (toward zero) at the point indicated by reference numeral 618 and as indicated by the portion 620 of the broken line 610. Thereafter, the first derivative of temperature for the conductive inclusion settles at the second rate of heat transfer, as indicated by the portion 622 of the broken line 610.

As discussed above, in instances in which only a few layers of laminate have been laid out, the kinks 506 and 518 of the temperature profiles for the normal regions and conductive inclusions may be perceivable in a suitable period of time for an inspection. In other instances in which many layers of laminate have been laid out, the kinks 506 and 518 may not be perceivable in a suitable period of time. Consequently, the changes of the first derivatives of temperature from the first rate of change (at portion 604) to the second rate of change (at portions 622 and 608) also would not be perceivable. However, the peak 614 of the first derivative of temperature for the conductive inclusion corresponding to the earlier kinks 512 and 514 would be perceivable relative to values of the first derivative of temperature for surrounding normal regions.

Figure 7A illustrates a chart 700 showing a calculated second derivative of the temperature of the top surface 111 of laminate 110 for the defect free (i.e., normal) region, indicated by the solid line 702, and to the region that has the conductive inclusion, discussed above with reference to Figure 5A, indicated by the broken line 720. As shown in Figure 7A, the second derivative of temperature for the normal region is zero along a first portion 704 of the solid line 702 until reaching a first peak 706 and then a second opposing peak 708 that corresponds with the kink 506 in the temperature plot shown in the chart 500 of Figure 5A and the rate of change trending from the first rate of change indicated by the portion 604 to the portion 608 (via portion 606) of the solid line 602 in Figure 6A. The second derivative of temperature for the region that includes the conductive inclusion results in similar peaks 726 and 728 that correspond to the kink 518 in the temperature plot of the chart 500 shown in Figure 5A and the rate of change trending from the first rate of change indicated by the portion 604 to the portion 622 (via portion 620) of the broken line 610 in Figure 6A. The second derivative of temperature for the region that includes the conductive inclusion also includes an additional set of peaks 722 and 724 that correspond with the kinks 512 and 514 of the temperature plot shown in Figure 5A and the rate of change changing temporarily from the first rate of change indicated by the portion 604 of the solid line 602 to the peak 614 rate of change and then returning to the first rate of change 604.

As discussed above, in instances in which only a few layers of laminate have been laid out, the kinks 506 and 518 of the temperature profiles for the normal regions and conductive inclusions may be perceivable in a suitable period of time for an inspection. In other instances in which many layers of laminate have been laid out, the kinks 506 and 518 may not be perceivable in a suitable period of time. Consequently, the peaks 706 and 708 for the normal regions and the peaks 726 and 728 for the conductive inclusion regions, corresponding to the kinks 506 and 518, may not be perceivable in a suitable period of time. However, the peaks 722 and 724 of the second derivative of temperature for the conductive inclusion, corresponding to the earlier kinks 512 and 514, would be perceivable relative to values of the first derivative of temperature for surrounding normal regions.

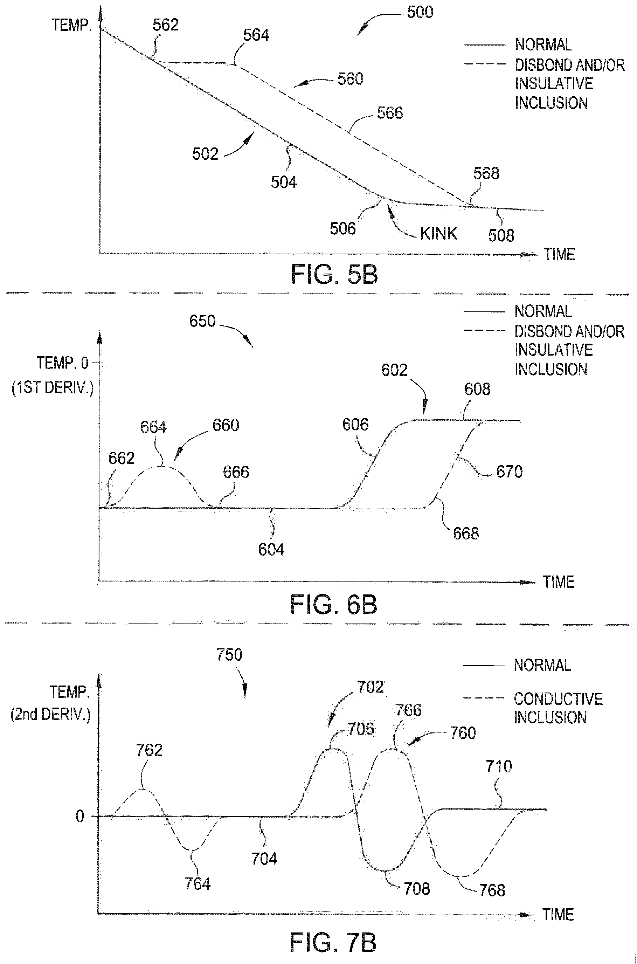

Figure 5B illustrates the differences in temperature of the top surface 111 of the laminate 110 over time for a defect-free region of the laminate and a region that has a disbond and/or an insulative inclusion. As explained above, an insulative inclusion is an inclusion (e.g., debris) that conducts heat less readily than the laminate layers. For example, a piece of backing paper caught between layers of the laminate could be an insulative inclusion. After a heat source, such as the heat source 214 shown in Figure 2, outputs a burst of energy directed toward the top surface 111 of the laminate 110, the temperature of the top surface 111 of the laminate 110 rises. The chart 550 in Figure 5B includes the solid line 502 indicating the normal (i.e., defect free) region of the laminate and a broken line 560 indicating the region that has a disbond and/or insulative inclusion. The left-hand ends of the solid line 502 and the broken line 560 indicate the temperature of the top surface 111 of the laminate 110 immediately after the burst of energy from the heat source 214 is applied to the top surface 111. Energy absorbed by the top surface 111 has caused the temperature of the top surface 111 to increase. After the initial increase in temperature of the top surface 111, the temperature of the top surface 111 begins to decrease in a linear manner as the heat energy applied to the top surface 111 travels deeper into the laminate via conduction (as indicated by first portion 504 of the solid line 502). The initial rate of heat transfer away from the top surface 111 via conduction is substantially the same for both the normal region and the region that includes the inclusion, as indicated by the initial overlap of the solid line 502 and the broken line 560.

Referring to the normal region, indicated by the solid line 502, as the heat energy reaches a bottom side of the laminate 110, the heat energy can travel no further through the laminate via conductance. Thereafter, the heat transfer away from the top surface 111 slows, as indicated by the kink 506 and the reduced-slope second portion 508 of the solid line 502..

Referring to the disbond and/or insulative inclusion region, indicated by the broken line 560, before the heat energy reaches the bottom side of the laminate 110, the heat energy encounters the disbond and/or insulative inclusion (e.g., the inclusion 122 shown in Figure 1C could be an insulative inclusion). The exemplary disbond and/or insulative inclusion provides additional mass that can act as a heat sink for the laminate such that heat from the top surface 111 can be transferred to the inclusion via conductance. However, because the exemplary disbond and/or insulative inclusion is less conductive than the surrounding layers of laminate, the top surface 111 of the laminate 110 over the disbond and/or insulative inclusion cools slower than surrounding regions of the top surface 111. This slower cooling of the top surface 111 is indicated by a first kink 562 of the broken line 560 as the broken line 560 deviates from the solid line 502. Over time, the heat transfer from the top surface 111 increases and the broken line 560 has a second kink 564. After the second kink 564, the rate of temperature change of the top surface 111 over the disbond and/or insulative inclusion is substantially equal to the rate of temperature change of the top surface 111 over normal regions of the laminate 110, as indicated by portion 504 of the solid line 502, as indicated by the portion 566 of the broken line 560. Because the disbond and/or insulative inclusion transfers heat away from the top surface 111 slowly, the heat reaches the back surface of the laminate 110 at the disbond and/or insulative inclusion slower than at surrounding normal regions. Thereafter, the heat transfer away from the top surface 111 over the disbond and/or insulative inclusion slows, as indicated by the kink 568 in the broken line, and eventually joins the second portion of the solid line 502.

For a laminate that is only three, four, or five layers thick, the kinks 506 and 568 in the temperature profiles for a normal region and a region that includes a disbond and/or insulative inclusion may be observable in a reasonable period of time. For example, the kinks 506 and 568 may occur within three to ten seconds after heat is applied to the top surface 111 of the laminate 110. However, in instances in which the laminate is thicker (e.g., including tens of layers, such as fifty layers, one hundred layers, or one hundred and fifty layers), the kinks 506 and 568 may not be observable in a suitable period of time for such an inspection. In such instances, the kink 562 and/or the kink 564 in the temperature profile of the top surface 111 over the disbond and/or insulative inclusion should be perceivable in a suitable period of time. The kink 506 in the temperature profile of the top surface 111 over normal regions does not need to be observed. For example, assuming that most of the laminate 110 is free from defects, the temperature profile over time would be substantially homogenous. However, if a particular region includes a disbond and/or insulative inclusion, that region would have a temperature profile that decreases at a slower rate (at the kink 562) than the defect free regions. Thus, observing a temperature profile decreasing at a slower rate than surrounding regions could indicate the presence of a disbond and/or insulative inclusion in the laminate 110.

Figure 6B illustrates a chart 650 showing a calculated first derivative of the temperature (i.e., a rate of change of temperature) of the top surface 111 of laminate 110 for the defect free (i.e., normal) region, indicated by the solid line 602, and of the region that has the disbond and/or insulative inclusion, discussed above with reference to Figure 5B, indicated by the broken line 660. As shown in Figure 6B, the first derivative of temperature for the normal region is a first rate of change, indicated by portion 604 of the solid line 602, which corresponds with the first portion 504 of the solid line 502 in Figure 5B. Since the temperature of the top surface 111 is decreasing, the values of the first derivative are negative. Stated differently, a zero value on the temperature axis of the chart 650 is above the illustrated solid line 602 and the broken line 610. When the temperature profile of the top surface 111 over the normal region reaches the kink 506 in Figure 5B, the first derivative of temperature for the normal region increases (toward zero), as indicated by the portion 606 of the solid line 602. Thereafter, the first derivative of temperature for the normal region settles at a second rate of heat transfer, as indicated by the portion 608 of the solid line 602. The first derivative of temperature for the region that includes the disbond and/or insulative inclusion diverges from the first derivative of temperature for the normal region twice. First, where the temperature profile of the top surface 111 for the disbond and/or insulative inclusion decreases slower than surround normal regions at the kinks 562 and 564 (in Figure 5B), the first derivative of temperature increases (toward zero) from the first rate of change (indicated by the portion 604 of the solid line 602) at the point indicated by reference numeral 662, reaches a peak at the point indicated by reference numeral 664, and then decreases (away from zero) at the point indicated by reference numeral 666. When the temperature profile of the top surface 111 over the disbond and/or insulative inclusion reaches the kink 568 in Figure 5B, the first derivative of temperature increases (toward zero) at the point indicated by reference numeral 668 and as indicated by the portion 670 of the broken line 660. Thereafter, the first derivative of temperature for the disbond and/or insulative inclusion settles at the second rate of heat transfer, as indicated by the portion 608 of the solid line 602.

As discussed above, in instances in which only a few layers of laminate have been laid out, the kinks 506 and 568 of the temperature profiles for the normal regions and disbond and/or insulative inclusions may be perceivable in a suitable period of time for an inspection. In other instances in which many layers of laminate have been laid out, the kinks 506 and 568 may not be perceivable in a suitable period of time. Consequently, the changes of the first derivatives of temperature from the first rate of change (at portion 604) to the second rate of change (at portion 608) also would not be perceivable. However, the peak 664 of the first derivative of temperature for the disbond and/or insulative inclusion corresponding to the earlier kinks 562 and 564 would be perceivable relative to values of the first derivative of temperature for surrounding normal regions.

Figure 7B illustrates a chart 750 showing a calculated second derivative of the temperature of the top surface 111 of laminate 110 for the defect free (i.e., normal) region, indicated by the solid line 702, and to the region that has the disbond and/or insulative inclusion, discussed above with reference to Figure 5B, indicated by the broken line 760. As shown in Figure 7B, the second derivative of temperature for the normal region is zero along a first portion 704 of the solid line 702 until reaching a first peak 706 and then a second opposing peak 708 that corresponds with the kink 506 in the temperature plot shown in the chart 550 of Figure 5B and the rate of change trending from the first rate of change indicated by the portion 604 to the portion 608 (via portion 606) of the solid line 602 in Figure 6B. The second derivative of temperature for the region that includes the disbond and/or insulative inclusion results in similar peaks 766 and 768 that correspond to the kink 568 in the temperature plot of the chart 550 shown in Figure 5B and the rate of change trending from the first rate of change indicated by the portion 604 to the portion 622 (via portion 620) of the broken line 660 in Figure 6B. The second derivative of temperature for the region that includes the disbond and/or insulative inclusion also includes an additional set of peaks 762 and 764 that correspond with the kinks 562 and 564 of the temperature plot shown in Figure 5B and the rate of change changing temporarily from the first rate of change indicated by the portion 604 of the solid line 602 to the peak 664 rate of change and then returning to the first rate of change of the solid line 604.

As discussed above, in instances in which only a few layers of laminate have been laid out, the kinks 506 and 568 of the temperature profiles for the normal regions and disbond and/or insulative inclusions may be perceivable in a suitable period of time for an inspection. In other instances in which many layers of laminate have been laid out, the kinks 506 and 568 may not be perceivable in a suitable period of time. Consequently, the peaks 706 and 708 for the normal regions and the peaks 766 and 768 for the disbond and/or insulative inclusion regions, corresponding to the kinks 506 and 568, may not be perceivable in a suitable period of time. However, the peaks 762 and 764 of the second derivative of temperature for the disbond and/or insulative inclusion, corresponding to the earlier kinks 562 and 564, would be perceivable relative to values of the first derivative of temperature for surrounding normal regions.

In instances in which the laminate 110 includes a neutral inclusion (e.g., an inclusion with the same heat transfer characteristics as the plies of the laminate, such as a scrap of another ply), such a neutral inclusion may be detectable if it occurs in the first several layers of a laminate. Such a neutral inclusion may include disbonds surrounding it. For example, the inclusion 122 shown in Figure 1C includes disbonds 124 (i.e., voids or air gaps) in regions immediately surrounding the inclusion. Such disbonds 124 may be detectable as discussed above with reference to Figures 5B, 6B, and 7B.

Referring again to Figure 2, in use, the heat transfer detection program 226 executing on the computer processor 222 could evaluate the temperature, first derivative of temperature, and/or second derivative of temperature for every pixel of every image stored in memory 224 from the digital thermographic camera 212. Pixels or regions of pixels that have different heat characteristics then surrounding pixels or regions could be identified as regions containing defects, and the defects could be addressed, as discussed above with reference to Figures 3 and 4. In aspects of the system 200 in which the digital thermographic camera 212 has a view of the entire laminate 110, each pixel of a digital thermographic image captured by the digital thermographic camera 212 corresponds to a particular region of the laminate 110. Thus, defects in the laminate 110, detected in pixels or regions of pixels in the digital thermographic images, can be located by finding the location(s) on the laminate 110 corresponding to the pixels that indicate the defect(s).

In various aspects of the system, the digital thermographic camera 212 may not capture an image of the entire laminate 110. In such aspects, the digital thermographic camera 212 and the heat source 214 may move in the directions of arrows 216 and/or 218 to capture images of different portions of the laminate 110. At least one aspect, the digital thermographic camera 212 and heat source 214 could continuously move while the heat source 214 applies heat to the top surface 111 of laminate 110 and the digital thermographic camera 212 captures digital thermographic images of the temperature of the top surface 111 of the laminate 110. In such aspects, the system 200 could map the pixels of the image to locations on the laminate 110 as described in United States Application Serial Number 14/614,198, filed on February 16, 2015, and entitled "system and method for high-speed FOD detection," the entire contents of which are incorporated by reference herein.

The system 200 could include one of several different combinations of digital thermographic camera 212 and heat source 214. For example, the digital thermographic camera 212 and heat source 214 could be included in a single unit such as the Thermoscope^{®} II manufactured by Thermal Wave Imaging. In at least one test, a similar thermal wave imaging system was able to detect an inclusion under two or three layers of uncured compacted composite sheets. As another example, the VoyageIR^{®} 2 thermal processing system by Thermal Wave Imaging combines the digital thermographic camera 212 and heat source 214 into a single unit and, in at least one test, was able to detect an inclusion under two or three layers of uncured compacted composite sheets. As another example, a ThermaCam^{™} SC640 infrared camera by FLIR Systems^{®} could be used as the digital thermographic camera 212, and could be used in combination with a heat source 212, such as a heat gun or lamp. In at least one test, such an infrared camera was able to detect an inclusion under one layer of uncured compacted composite sheet. As yet another example, an E40 infrared camera by FLIR Systems^{®} could be used as the digital thermographic camera 212, and could be used in combination with a heat source 212, such as a heat gun or lamp. In at least one test, such an infrared camera was able to detect an inclusion under one layer of uncured compacted composite sheet.

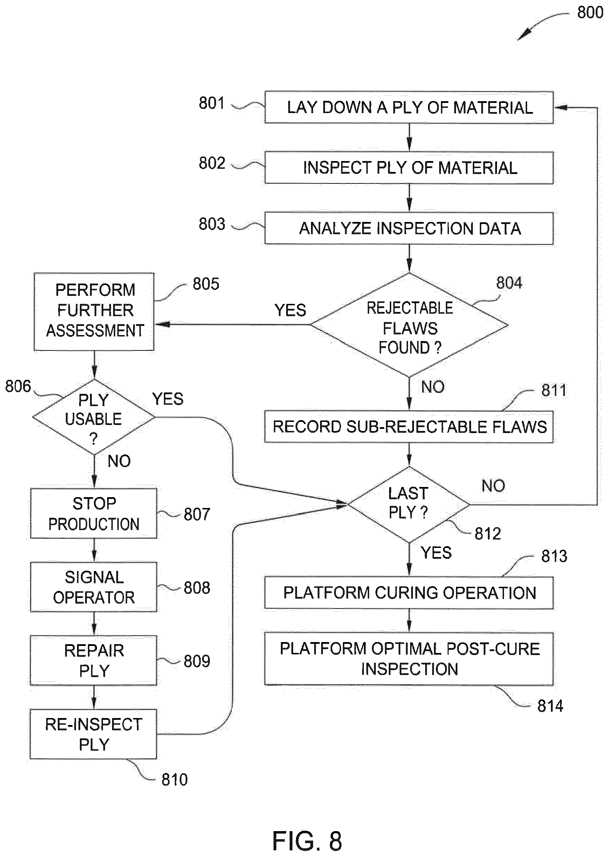

Figure 8 is a flow chart of a process 800 for detecting a defect according to another aspect of the disclosure. Process 800 is similar to process 300. However, process 800 performs flaw detection analysis after each ply of material is laid down. Therefore, process 800 allows defects to be identified before additional plies are positioned thereon, thus resulting in more simplified repair processes. Additionally, in instances where a defect is determined to be beyond repair, the ply (or entire workpiece) can be scrapped, rather than completing layup of additional plies. In such an example, materials are not wasted on unusable plies or workpieces, thereby resulting in cost savings.

Process 800 begins at block 801. At block 801, an uncured ply of material, such as a composite material described herein, is laid down. The uncured ply of material may be one of multiple plies of a workpiece, such as an aircraft part or component. At block 802, the ply of material is inspected, for example, by infrared thermography. Infrared thermography inspection includes subjecting the uncured ply to a burst of thermal energy, and capturing at least one digital thermographic image of the surface of the uncured ply, as described above. In such an example, inspection may be a full line scan or a flash scan. To facilitate data collection, it is contemplated that a digital thermographic camera 212 (shown in Figure 2) for imaging the thermally-subjected ply may be positioned on a fabrication head configured to lay the uncured ply of material.

Optionally, other optical inspections may be performed during block 802. The optical inspections include one or more of laser line scans, high-resolution video, or other non-contact sensing of the ply. The optical data and the infrared thermography data are subsequently stored in a memory. In one example, the optical data is correlated to the infrared thermography data based on, for example, part geometry. To facilitate correlation, a CAD model or other digital representation may be utilized.

In block 803, inspection data captured during block 802 is analyzed to determine the presence of defects in or immediately beneath the uncured ply laid down in block 801. The analysis in block 803 includes comparing captured inspection data to rejection criteria, such as baseline data, to facilitate identification of inspection data from block 802 that is out of a desired range. The baseline data may include inspection data of previous plies of known acceptable quality, or may include operator-input data or ranges. In one example, the baseline data may include thermal data to which captured data (e.g., data from block 802) is compared. By comparing the data of block 802 to the rejection criteria, defects, foreign objects and debris (FOD), missing plies, geometry-tolerance errors, and the like can be identified. It is contemplated that the analysis of block 803 may occur automatically, that is, without specific operator input.

In block 804, a determination is made by a controller as to whether a rejectable flaw (e.g., a flaw outside of a predetermined acceptable quality range) is identified in block 803. In one example, a rejectable flaw is determined to exist if a threshold variance is exceeded when comparing baseline data to captured data. If the determination in block 804 is affirmative, process 800 proceeds to block 805, in which additional assessment is performed. In block 805, a model-based assessment is performed to determine the effect of the rejectable flaw. The model-based assessment determines the effect of the rejectable flaw with respect to structure, thermal, adhesive, or other qualities. In one example, it is contemplated that model-based assessments may be catalogued, and used for subsequent assessments, when similar, thereby reducing processing time by mitigating computations from the analysis.

After block 805, a determination is made in block 806 as to whether the ply (or workpiece) having the rejectable flaw is usable as-is. If the ply or workpiece having the rejectable flaw is unusable, production is stopped in block 807. In block 808, an operator is signaled that production has stopped and/or that an unusable ply has been identified. In block 809, the rejectable flaw is repaired. Example repair processes are discussed above, but are not to be considered limiting. In one example, flaw repair may include FOD removal, ply removal, defect repair, and the like. Because the rejectable flaw is repaired before laying additional plies on the workpiece, the rejectable flaw is relatively easy to access, thereby simplifying the repair processes and resulting in a less time-consuming or less costly repair. Additionally, in the repair operation of block 809, an operator can rely on information obtained in block 802 and 803, thereby further simplifying the repair process by providing the operator with more accurate and more complete information for completing the repair. For example, precise geometric coordinates of the flaw may be provided, thereby aiding in identification and/or repair of the flaw by the repairing operator.

After the repair processes have been completed, the repaired ply is re-inspected in block 810. The re-inspection processes may be as thorough as the inspection of block 802, or the re-inspection may be limited reinspection that focuses only on the repaired area, or that otherwise performs limited analysis, such as less than all inspections performed in block 802. Upon completion of operation 810, the inspection data for the workpiece/ply obtained in block 802 is updated to include the reinspection data of block 810 in order to provide comprehensive monitoring of the ply.

Returning to block 804, if no rejectable flaws are found, then process 800 proceeds to block 811. In block 811, any identified sub-rejectable flaws (from block 803) are recorded in a memory by a controller or other processing unit. Sub-rejectable flaws include flaws outside of a predetermined quality range, but not rising to a level of a rejectable flaw. Recording of one or more of the location, type, and extent of the non-rejectable flaw facilitates tracking and monitoring of the non-rejectable flaw during additional processing, such as the placement of additional plies on the workpiece. Thus, should the non-rejectable flaw become (or generate) a rejectable flaw during future processing, the rejectable flaw can more easily be identified. In one example, it is contemplated that one or more process conditions of future processes (such as laying of future plies) are adjusted, manually or automatically, to compensate and/or mitigate flaw exacerbation of sub-rejectable flaws. Thus, it is possible to reduce the likelihood that sub-rejectable flaws become rejectable flaws. In addition, if a controller detects of pattern of reoccurring rejectable or sub-rejectable flaws on workpieces, the controller may facilitate an adjustment to an upstream production process to mitigate or eliminate reoccurring flaw on future plies or workpieces.

In block 812, a determination is made as to whether a final ply has been laid. If the final ply has not been laid on the workpiece, process 800 returns to block 801. If the final ply has been laid, process 800 proceeds to block 813, and a curing operation is performed on the one or more plies of the workpiece. The curing operation of block 812 is similar to the curing operations of block 312 (shown in Figure 3) and/or block 416 (shown in Figure 4) discussed above. After curing, an optional post-cure inspection may be performed in block 814. The post-cure inspection may be a complete inspection, or a partial inspection, such as only on a selected area. In one example, the selected area is an area in which a rejectable flaw or a sub-rejectable flaw has been previously identified. Whether or not a post-cure inspection is performed may be dictated by the number of rejectable flaws and/or non-rejectable flaws identified during processing, among other factors.

Figure 8 illustrates one example of a process 800. However, other examples and modifications are contemplated. In one example, it is contemplated that inspection data acquired in block 802 for a ply in which no rejectable flaws or sub-rejectable flaws are found may be used to update the baseline data used in block 803, for example, by combining the data acquired with block 802 and determining an average, or alternatively, but adjusting a range of the baseline data which indicates acceptable processing. Thus, the baseline data becomes refined and more accurate (via a larger sample size) as processing proceeds. In such an example, it is contemplated that the baseline data may be correlated to a CAD model. In another example, all data collected for plies which remain in the workpiece (including plies having sub-rejectable flaws) is stored to facilitate trend analysis, tool/part redesign, process modifications, and the like.

## Claims

1. A method (800) for detecting a defect in a workpiece, wherein the workpiece comprises a plurality of plies;
wherein, for each of the plurality of plies, the following steps are conducted:
laying (801) a ply of material;
applying (802) thermal energy to a surface of the ply of material;
capturing (802) at least one digital thermographic image of the surface of the ply of material after applying the thermal energy;
using a computer processor, analyzing (803) at least one heat characteristic of regions of the ply of material in the at least one digital thermographic image to identify defect regions in which the at least one heat characteristic is different than baseline data, wherein analyzing the at least one heat characteristic of regions of the ply of material in the at least one digital thermographic image comprises determining (804) whether a rejectable flaw exists and determining (811) whether a sub-rejectable flaw exists;
wherein, when it is determined that a rejectable flaw exists, the following steps are performed:
halting (807) processing;
outputting (808) an alert;
repairing (809) the rejectable flaw; and
resuming processing;
wherein, when it is determined that a sub-rejectable flaw exists, the following steps are performed:
recording one or more of the location, type, and extent of the sub-rejectable flaw; and
resuming processing;
wherein the method further comprises:
determining (812) whether a final ply of the workpiece has been laid; and
laying (801) a further ply of material on the ply of material, if the ply (112) is not the final ply of the workpiece.

2. The method of claim 1, further comprising curing (813) the ply of material and the further ply of material after laying the further ply of material.

3. The method of claim 1 or 2, wherein analyzing (803) at least one heat characteristic in the at least one digital thermographic image comprises analyzing at least one heat characteristic of every pixel in the at least one digital thermographic image.

4. The method of any of claims 1 to 3, wherein the at least one heat characteristic comprises at least one of respective temperatures of the regions, respective first derivative rates of change of temperature of the regions over a plurality of digital thermographic images, and respective second derivative rates of change of temperature of the regions over a plurality of digital thermographic images, and wherein the at least one heat characteristic of a subject region is different than surrounding regions if a peak of a second derivative rate of change of temperature for the subject region differs in time from a peak of a second derivative rate of change of temperature of at least one surrounding regions by more than a threshold amount.

5. The method of any of claims 1 to 4, further comprising:
determining whether the further ply of material includes a defect; and
updating, if it is detemined that the further ply of material does not include a defect, the baseline data based on a digital thermographic image of a surface of the further ply of material.

6. The method of any of claims 1 to 5, further comprising reinspecting (810) the ply of material after resuming processing, wherein reinspecting comprises:
applying thermal energy to a surface of the ply of material; and
capturing at least one digital thermographic image of the surface of the ply of material after applying the thermal energy.

7. The method of any of claims 1 to 6, wherein the alert includes an indication of a location of the defect region on the ply (112).

8. The method of any of claims 1 to 7, wherein sub-rejectable flaws include flaws outside of a predetermined quality range, but not rising to a level of a rejectable flaw.

9. A system for detecting a defect in a workpiece, wherein the workpiece comprises a plurality of plies, comprising:
a work surface (104) for arranging and compacting uncured composite layers;
a heat source (214) arranged relative to the surface and operable to provide thermal energy to a surface of a ply of material on the work surface (104);
a thermographic digital camera (212) arranged relative to the surface (104) and operable to capture at least one digital thermographic image of the surface of the ply of material;
a computer processor (222) in communication with the thermographic digital camera (212) and the heat source (214); and
a computer memory (224) containing a program (226) that, when executed on the computer processor (222), performs, for each of the plurality of plies, an operation for processing data, comprising:
applying (802) thermal energy to a surface of the ply of material;
capturing (802) at least one digital thermographic image of the surface of the ply (112) of material after applying the thermal energy;
analyzing (803) at least one heat characteristic of regions of the ply of material in the at least one digital thermographic image to identify defect regions in which the at least one heat characteristic is different than baseline data, wherein analyzing the at least one heat characteristic of regions of the ply of material in the at least one digital thermographic image comprises determining (804) whether a rejectable flaw exists and determining (811) whether a sub-rejectable flaw exists;
wherein, when it is determined that a rejectable flaw exists, the following steps are performed:
halting (807) processing;
outputting (808) an alert;
repairing (809) the rejectable flaw; and
resuming processing;
wherein, when it is determined that a sub-rejectable flaw exists, the following steps are performed:
recording one or more of the location, type, and extent of the sub-rejectable flaw; and
resuming processing;
wherein the program (226), when executed on the computer processor (222), performs an additional operation for processing data, comprising:
determining whether a final ply of the workpiece has been laid; and
laying (801) a further ply of material on the ply of material after resuming processing, if the ply is not the final ply of the workpiece.

10. The system of claim 9, wherein the program (226), when executed on the computer processor (222), performs an additional operation for processing data, comprising, upon receiving an indication of a repair being made to the defect region:
outputting a second signal to the heat source (214) to apply a second thermal energy to the surface of the ply;
capturing a second at least one digital thermographic image of the surface of the ply after applying the second thermal energy;
analyzing at least one heat characteristic of regions of the ply in the second at least one digital thermographic image to identify defect regions in which the at least one heat characteristic is different than surrounding regions.

11. The system of claim 9 or 10, wherein analyzing (803) at least one heat characteristic of the ply in the at least one digital thermographic image to identify regions in which the at least one heat characteristic is different than surrounding regions comprises analyzing at least one heat characteristic of every pixel in the at least one digital thermographic image.

12. The system of any of claims 9 to 11, wherein the at least one heat characteristic of the ply comprises at least one of respective temperatures of the regions, respective first derivative rates of change of temperature of the regions over a plurality of digital thermographic images, and respective second derivative rates of change of temperature of the regions over a plurality of digital thermographic images.

13. The system of claim 12, wherein the at least one heat characteristic of a subject region is different than surrounding regions if a peak of a second derivative rate of change of temperature for the subject region differs in time from a peak of a second derivative rate of change of temperature of at least one surrounding regions by more than a threshold amount.

14. The system of any of claims 9 to 13, wherein sub-rejectable flaws include flaws outside of a predetermined quality range, but not rising to a level of the rejectable flaw.

15. The system of any of claims 8 to 13, wherein the alert includes an indication of a location of the defect region.

## Patentansprüche

1. Verfahren (800) zum Detektieren eines Defekts in einem Werkstück, wobei das Werkstück eine Mehrzahl von Lagen aufweist;
wobei, für jede der Mehrzahl von Lagen, die folgenden Schritte durchgeführt werden:
Ablegen (801) einer Materiallage;
Beaufschlagen (802) von thermischer Energie auf einer Oberfläche der Materiallage;
Erfassen (802) von mindestens einem digitalen thermografischen Bild der Oberfläche der Materiallage nach dem Beaufschlagen der thermischen Energie;
unter Nutzung eines Computerprozessors, Analysieren (803) mindestens einer Wärmecharakteristik von Bereichen der Materiallage in dem mindestens einen digitalen thermografischen Bild zum Identifizieren von Defektbereichen, in denen die mindestens eine Wärmecharakteristik verschieden von Basisdaten ist, wobei das Analysieren der mindestens einen Wärmecharakteristik von Bereichen der Materiallage in dem mindestens einen digitalen thermografischen Bild das Bestimmen (804) aufweist, ob ein zurückweisbarer Fehler vorliegt und Bestimmen (811) aufweist, ob ein unter-zurückweisbarer Fehler vorliegt;
wobei, wenn bestimmt wird, ein zurückweisbarer Fehler vorliegt, die folgenden Schritte durchgeführt werden:
Anhalten (807) der Verarbeitung;
Ausgeben (808) eines Alarms;
Reparieren (809) des zurückweisbaren Fehlers; und
Wiederaufnehmen des Verarbeitens;
wobei, wenn bestimmt wird, dass ein unter-zurückweisbarer Fehler vorliegt, die folgenden Schritte durchgeführt werden:
Aufzeichnen von einem oder mehreren von dem Ort, der Art und dem Ausmaß des unter-zurückweisbaren Fehlers; und
Wiederaufnehmen des Verarbeitens;
wobei das Verfahren des Weiteren aufweist:
Bestimmen (812), ob eine letzte Lage des Werkstücks abgelegt worden ist; und
Ablegen (801) einer weiteren Materiallage auf die Materiallage, falls die Lage (112) nicht die letzte Lage des Werkstücks ist.

2. Verfahren nach Anspruch 1, des Weiteren mit dem Aushärten (813) der Materiallage und der weiteren Materiallage nach dem Ablegen der weiteren Materiallage.

3. Verfahren nach Anspruch 1 oder 2, wobei das Analysieren (803) der mindestens einen Wärmecharakteristik in dem mindestens einen digitalen thermografischen Bild das Analysieren mindestens einer Wärmecharakteristik von jedem Pixel in dem mindestens einen digitalen thermografischen Bild aufweist.

4. Verfahren nach einem der Ansprüche 1 bis 3, wobei die mindestens eine Wärmecharakteristik mindestens einer von jeweiligen Temperaturen der Bereiche, der jeweilige ersten Ableitung von Änderungen der Temperaturen der Bereiche über eine Mehrzahl von digitalen thermografischen Bildern und jeweiligen zweiten Ableitungen der Änderung der Temperatur der Bereiche über eine Mehrzahl von digitalen thermografischen Bildern aufweist, und wobei die mindestens eine Wärmecharakteristik eines bestimmten Bereichs verschieden von umgebenden Bereichen ist, falls eine Spitze einer zweiten Ableitung einer Änderung der Temperatur für den bestimmten Bereich über die Zeit von einer Spitze einer zweiten Ableitung einer Änderung der Temperatur von mindestens einem umgebenden Bereich um mehr als eine Schwellwertmenge abweicht.

5. Verfahren nach einem der Ansprüche 1 bis 4, des Weiteren mit:
Bestimmen, ob die weitere Materiallage ein Defekt aufweist; und
Aktualisieren, falls es bestimmt ist, dass die weitere Materiallage kein Defekt aufweist, der Basisdaten basierend auf einem digitalen thermografischen Bild einer Oberfläche der weiteren Materiallage.

6. Verfahren nach einem der Ansprüche 1 bis 5, des Weiteren mit dem Wiederinspizieren (810) der Materiallage nach dem Wiederaufnehmen der Verarbeitung, wobei das Wiederinspizieren aufweist:
Beaufschlagen von thermischer Energie auf einer Oberfläche der Materiallage; und
Erfassen mindestens eines digitalen thermografischen Bilds der Oberfläche der Materiallage nach dem Beaufschlagen der thermischen Energie.

7. Verfahren nach einem der Ansprüche 1 bis 6, wobei der Alarm eine Angabe eines Orts des defekten Bereichs auf der Lage (112) aufweist.

8. Verfahren nach einem der Ansprüche 1 bis 7, wobei unter-zurückweisbare Fehler Fehler aufweisen, die außerhalb eines vorbestimmten Qualitätsbereichs liegen, aber nicht auf ein Niveau eines zurückbaren Fehlers steigen.

9. System zum Detektieren eines Defekts in einem Werkstück, wobei das Werkstück eine Mehrzahl von Lagen aufweist, mit:
einer Arbeitsfläche (104) zum Anordnen und Zusammendrücken unausgehärteter Verbundwerkstoffschichten;
einer Wärmequelle (214), die relativ zu der Fläche angeordnet ist und dazu betreibbar ist, thermische Energie auf einer Oberfläche einer Materiallage auf der Arbeitsfläche (104) bereitzustellen;
einer thermografischen digitalen Kamera (212), die relativ zu der zu der Fläche (104) angeordnet ist und dazu betreibbar ist, mindestens ein digitales thermografisches Bild von der Oberfläche der Materiallage zu erfassen;
einem Computerprozessor (222) in Kommunikation mit der thermografischen digitalen Kamera (212) und der Wärmequelle (214); und
einem Computerspeicher (224), der ein Programm (226) enthält, das, wenn es auf dem Computerprozessor (222) ausgeführt wird, für jede der Mehrzahl von Lagen im Betrieb zum Verarbeiten von Daten ausführt, mit:
Beaufschlagen (802) von thermischer Energie auf einer Oberfläche der Materiallage;
Erfassen (802) mindestens eines digitalen thermografischen Bilds der Oberfläche der Materiallage (112) nach dem Beaufschlagen der thermischen Energie;
Analysieren (803) mindestens einer Wärmecharakteristik von Bereichen der Materiallage in dem mindestens einen digitalen thermografischen Bild zum Identifizieren von Defektbereichen, in denen die mindestens eine Wärmecharakteristik verschieden von Basisdaten ist, wobei das Analysieren der mindestens einen Wärmecharakteristik von Bereichen der Materiallage in dem mindestens einen digitalen thermografischen Bild das Bestimmen (804) aufweist, ob ein zurückweisbarer Fehler vorliegt und das Bestimmen (811) aufweist, ob ein unter-zurückweisbarer Fehler vorliegt;
wobei, wenn bestimmt wird, dass ein zurückweisbarer Fehler vorliegt, die folgenden Schritte durchgeführt werden:
Anhalten (807) der Verarbeitung;
Ausgeben (808) eines Alarms;
Reparieren (809) des zurückweisbaren Fehlers; und
Wiederaufnehmen des Verarbeitens;
wobei, wenn bestimmt wird, dass ein unter-zurückweisbarer Fehler vorliegt, die folgenden Schritte durchgeführt werden:
Aufzeichnen von einem oder mehreren des Orts, der Art und des Ausmaßes des unter-zurückweisbaren Fehlers; und
Wiederaufnehmen der Verarbeitung;
wobei das Programm (226), wenn auf dem Computerprozessor (222) ausgeführt wird, einen zusätzlichen Betrieb zum Verarbeiten von Daten durchführt, aufweisend:
Bestimmen, ob eine letzte Lage des Werkstücks abgelegt worden ist; und
Ablegen (801) einer weiteren Materiallage auf die Materiallage nach dem Wiederaufnehmen der Verarbeitung, falls die Lage nicht die letzte Lage des Werkstücks ist.

10. System nach Anspruch 9, wobei das Programm (226), wenn es auf dem Computerprozessor (222) ausgeführt wird, einen zusätzlichen Betrieb zum Verarbeiten von Daten durchführt, aufweisend, beim Empfangen einer Angabe, dass eine Reparatur an dem Defektbereich durchzuführen ist:
Ausgeben eines zweiten Signals an die Wärmequelle (214) zum Beaufschlagen einer zweiten thermischen Energie auf die Fläche der Lage;
Erfassen eines zweiten, mindestens einen digitalen thermografischen Bild der Oberfläche der Materiallage nach dem Beaufschlagen der zweiten thermischen Energie;
Analysieren mindestens einer Wärmecharakteristik von Bereichen der Lage in den zweiten mindestens einen digitalen thermografischen Bild zum Identifizieren von Defektbereichen, in denen die mindestens eine Wärmecharakteristik verschieden von umgebenden Bereichen ist.

11. System nach Anspruch 9 oder 10, wobei das Analysieren (803) der mindestens einen Wärmecharakteristik der Lage in dem mindestens einen digitalen thermografischen Bild zum Identifizieren von Bereichen, in denen die mindestens eine Wärmecharakteristik verschieden von umgebenden Bereichen ist, das Analysieren mindestens einer Wärmecharakteristik von jedem Pixel in dem mindestens einen digitalen thermografischen Bild aufweist.

12. System nach einem der Ansprüche 9 bis 11, wobei die mindestens eine Wärmecharakteristik der Lage mindestens eines von jeweiligen Temperaturen der Bereiche, der jeweiligen ersten Ableitungen mit Änderung von Temperaturen der Bereiche über eine Mehrzahl von digitalen thermografischen Bildern und jeweilige zweite Ableitungen von Änderungen der Temperaturen der Bereiche über eine Mehrzahl von digitalen thermografischen Bildern aufweist.

13. System nach Anspruch 12, wobei die mindestens eine Wärmecharakteristik eines spezifischen Bereichs verschieden von umgebenden Bereichen ist, falls eine Spitze einer zweiten Ableitung von Änderungen von Temperatur für bestimmte Bereiche, über die Zeit von einer Spitze einer zweiten Ableitung von Änderungen der Temperatur von mindestens einem umgebenden Bereich um mehr als eine Schwellwertmenge abweicht.

14. System nach einem der Ansprüche 9 bis 13, wobei unter-zurückweisbare Fehler Fehler außerhalb eines vorbestimmten Qualitätsbereichs aufweisen, aber nicht auf ein Niveau des zurückweisbaren Fehlers ansteigen.

15. System nach einem der Ansprüche 8 bis 13, wobei der Alarm eine Angabe eines Orts des defekten Bereichs aufweist.

## Revendications

1. Procédé (800) pour détecter un défaut dans une pièce de travail, la pièce de travail comprenant une pluralité de plis ;
pour chaque pli de la pluralité de plis, les étapes suivantes sont menées :
pose (801) d'un pli de matériau ;
application (802) d'énergie thermique à une surface du pli de matériau ;
capture (802) d'au moins une image thermographique numérique de la surface du pli de matériau après l'application de l'énergie thermique ;
à l'aide d'un processeur informatique, analyse (803) d'au moins une caractéristique de chaleur de régions du pli de matériau dans l'au moins une image thermographique numérique pour identifier des régions de défaut dans lesquelles l'au moins une caractéristique de chaleur est différente des données de ligne de base, l'analyse de l'au moins une caractéristique de chaleur de régions du pli de matériau dans l'au moins une image thermographique numérique comprenant le fait de déterminer (804) si un défaut à rejeter existe et de déterminer (811) si une sous-faille rejetable existe ;
où, lorsqu'il est déterminé qu'une faille rejetable existe, les étapes suivantes sont réalisées :
arrêt (807) du traitement ;
sortie (808) d'une alerte ;
réparation (809) de la faille rejetable ; et
reprise du traitement ;
où, lorsqu'il est déterminé qu'une sous-faille rejetable existe, les étapes suivantes sont réalisées :
enregistrement d'un ou de plusieurs éléments parmi l'emplacement, le type, et l'étendue de la sous-faille rejetable ; et
reprise du traitement ;
le procédé comprenant en outre :
le fait de déterminer (812) si un pli final de la pièce de travail a été posé ; et
pose (801) d'un pli supplémentaire de matériau sur le pli de matériau, si le pli (112) n'est pas le pli final de la pièce de travail.

2. Procédé selon la revendication 1, comprenant en outre le durcissement (813) du pli de matériau et du pli supplémentaire de matériau après la pose du pli supplémentaire de matériau.

3. Procédé selon la revendication 1 ou la revendication 2, où l'analyse (803) d'au moins une caractéristique de chaleur dans l'au moins une image thermographique numérique comprend l'analyse d'au moins une caractéristique de chaleur de chaque pixel dans l'au moins une image thermographique numérique.

4. Procédé selon l'une quelconque des revendications 1 à 3, où l'au moins une caractéristique de chaleur comprend au moins un élément parmi des températures respectives des régions, des premiers taux dérivés respectifs de changement de température des régions sur une pluralité d'images thermographiques numériques, et des deuxièmes taux dérivés respectifs de changement de température des régions sur une pluralité d'images thermographiques numériques, et l'au moins une caractéristique de chaleur d'une région sujette est différente des régions environnantes si un pic d'un deuxième taux de changement de température pour la région sujette diffère dans le temps d'un pic d'un deuxième taux dérivé de changement de température d'au moins une région environnante de plus d'une quantité seuil.

5. Procédé selon l'une quelconque des revendications 1 à 4, comprenant en outre :
le fait de déterminer si le pli supplémentaire de matériau comprend un défaut ; et
la mise à jour, s'il est déterminé que le pli supplémentaire de matériau n'inclut pas de défaut, des données de ligne de base sur la base d'une image thermographique numérique d'une surface du pli supplémentaire de matériau.

6. Procédé selon l'une quelconque des revendications 1 à 5, comprenant en outre la réinspection (810) du pli du matériau après la reprise de traitement, la réinspection comprenant :
l'application d'énergie thermique à une surface du pli de matériau ; et
la capture d'au moins une image thermographique numérique de la surface du pli de matériau après l'application de l'énergie thermique.

7. Procédé selon l'une quelconque des revendications 1 à 6, où l'alerte inclut une indication d'un emplacement de la région de défaut sur le pli (112).

8. Procédé selon l'une quelconque des revendications 1 à 7, où des failles rejetables incluent des failles à l'extérieur d'une plage de qualité prédéterminée, mais ne s'élèvent pas à un niveau d'une faille rejetable.

9. Système pour détecter un défaut dans une pièce de travail, la pièce de travail comprenant une pluralité de plis, comprenant :
une surface de travail (104) pour agencer et compacter des couches composites non durcies ;
une source de chaleur (214) agencée relativement à la surface et utilisable pour fournir de l'énergie thermique à une surface d'un pli de matériau sur la surface de travail (104) ;
une caméra numérique thermographique (212) agencée relativement à la surface (104) et utilisable pour capturer au moins une image thermographique numérique de la surface du pli de matériau ;
un processeur informatique (222) en communication avec la caméra numérique thermographique (212) et la source de chaleur (214) ; et
une mémoire informatique (224) contenant un programme (226) qui, lorsqu'il est exécuté sur le processeur informatique (222), réalise, pour chaque pli de la pluralité de plis, une opération de traitement de données, comprenant :
l'application (802) d'énergie thermique à une surface du pli de matériau ;
la capture (802) d'au moins une image thermographique numérique de la surface du pli (112) de matériau après l'application de l'énergie thermique ;
l'analyse (803) d'au moins une caractéristique de chaleur de régions du pli de matériau dans l'au moins une image thermographique numérique pour identifier des régions de défaut dans lesquelles l'au moins une caractéristique de chaleur est différente des données de ligne de base, l'analyse de l'au moins une caractéristique de chaleur de régions du pli de matériau dans l'au moins une image thermographique numérique comprenant le fait de déterminer (804) si une faille rejetable existe et de déterminer (811) si une sous-faille rejetable existe ;
où, lorsqu'il est déterminé qu'une faille rejetable existe, les étapes suivantes sont réalisées :
arrêt (807) du traitement ;
sortie (808) d'une alerte ;
réparation (809) de la faille rejetable ; et
reprise du traitement ;
où, lorsqu'il est déterminé qu'une sous-faille rejetable existe, les étapes suivantes sont réalisées :
enregistrement d'un ou de plusieurs éléments parmi l'emplacement, le type, et l'étendue de la sous-faille rejetable ; et
reprise du traitement ;
où le programme (226), lorsqu'il est exécuté sur le processeur informatique (222), réalise une opération supplémentaire pour traiter des données, comprenant :
le fait de déterminer si un pli final de la pièce de travail a été posé ; et
la pose (801) d'un pli supplémentaire de matériau sur le pli de matériau après la reprise du traitement, si le pli n'est pas le pli final de la pièce de travail.

10. Système selon la revendication 9, où le programme (226), lorsqu'il est exécuté sur le processeur informatique (222), réalise une opération supplémentaire pour traiter des données, comprenant, lors de la réception d'une indication d'une réparation réalisée sur la région de défaut :
la sortie d'un deuxième signal à la source de chaleur (214) pour appliquer une deuxième énergie thermique à la surface du pli ;
la capture d'une deuxième au moins une image thermographique numérique de la surface du pli de matériau après l'application de la deuxième énergie thermique ;
l'analyse d'au moins une caractéristique de chaleur de régions de la couche dans la deuxième au moins une image thermographique numérique pour identifier des régions de défaut dans lesquelles l'au moins une caractéristique de chaleur est différente des régions environnantes.

11. Système selon la revendication 9 ou la revendication 10, dans lequel l'analyse (803) d'au moins une caractéristique de chaleur du pli dans l'au moins une image thermographique numérique pour identifier des régions dans lesquelles l'au moins une caractéristique de chaleur est différente des régions environnantes comprend l'analyse d'au moins une caractéristique de chaleur de chaque pixel dans l'au moins une image thermographique numérique.

12. Système selon l'une quelconque des revendications 9 à 11, où l'au moins une caractéristique de chaleur du pli comprend au moins un élément parmi des températures respectives des régions, des premiers taux dérivés respectifs de changement de température des régions sur une pluralité d'images thermographiques numériques, et des deuxièmes taux dérivés respectifs de changement de température des régions sur une pluralité d'images thermographiques numériques.

13. Système selon la revendication 12, où l'au moins une caractéristique de chaleur d'une région sujette est différente des régions environnantes si un pic d'un deuxième taux de changement de température pour la région sujette diffère dans le temps d'un pic d'un deuxième taux de changement de température d'au moins une région environnante de plus d'une quantité seuil.

14. Système selon l'une quelconque des revendications 9 à 13, où des sous-failles rejetables incluent des failles à l'extérieur d'une plage de qualité prédéterminée, mais ne s'élèvent pas à un niveau de faille rejetable.

15. Système selon l'une quelconque des revendications 8 à 13, où l'alerte inclut une indication d'un emplacement de la région de défaut sur le pli.
